# EUROPEAN PATENT APPLICATION

(11) **EP 2 353 599 A1**
(43) Date of publication of application: **10.08.2011**
(21) Application number: 10000896.0
(22) Date of filing: 28.01.2010
(51) Int. Cl.: A61K 31/4709, A61P 25/00, A61P 19/00, A61P 37/00, A61P 35/00

(54) **8-hydroxyquinolines as inhibitors of cathepsin B**

(71) Applicant: University of Ljubljana, 1000 Ljubljana (SI)
(72) Inventor: Mirkovic, Bojana, 8212 Velika Loka (SI); Turk, Samo, 1296 Sentvid Pri Sticni (SI)
(74) Representative: Schmidt, Karsten

(57) **Abstract**

This invention relates to the compounds or a pharmaceutical acceptable salts, hydrates or solvates thereof, which are inhibitors of cysteine proteases, in particular of cathepsin B. Compounds of the invention are useful in the treatment of diseases in which cathepsin B is implicated, such as cancer, rheumatoid arthritis, osteoarthritis, osteoporosis, pancreatitis, immune and neurodegenerative diseases, e.g. Alzheimer's disease.

## Description

### FIELD OF THE INVENTION

The present invention relates to novel 8-hydroxyquinoline cysteine protease inhibitors. In particular, this invention relates to 8-hydroxyquinolines as the inhibitors of cysteine proteases of the papain superfamily, and yet more particularly cysteine proteases of the cathepsin (C1) family. In the most preferred embodiment, this invention relates to compounds which inhibit cathepsin B. Such compounds are particularly useful in the treatment of diseases in which cysteine proteases are implicated, especially diseases such as cancer, rheumatoid arthritis, osteoarthritis, osteoporosis, pancreatitis, immune and neurodegenerative diseases, e.g. Alzheimer's disease.

### BACKGROUND OF THE INVENTION

Cathepsin B is a lysosomal cysteine protease which belongs to the papain superfamily of cysteine proteases. The mature form of the enzyme is comprised of two domains, left and right. Between them the active site cleft is located which is at the upper end occluded by a 21 amino acid insertion termed the occluding loop. The latter is flexible and can adopt two different conformations enabling cathepsin B to cleave its substrates as an endopeptidase and also as an exopeptidase. The endopeptidase conformation is associated with the endopeptidase activity by which the enzyme cleaves its substrates in the middle of the polypeptide chain, whilst in the exopeptidase conformation the enzyme possesses exopeptidase activity by which it cleaves dipeptides from the C-terminal end of the polypeptide chain. The endopeptidase activity is associated with the degradation of proteins of extracellular matrix, a process which is required for tumour invasion and metastasis. The latter activity prevails at neutral pH values which are associated with extracellular environment. On the other hand, at lower pH values cathepsin B cleaves its substrates as an exopeptidase, which is an activity primarily associated with the enzyme's physiological environment within lysosomes.

It was long believed that the physiological function of cathepsin B was solely the protein turnover within lysosomes. However, it was discovered that cathepsin B also participates in the liberation of thyroid hormones from thyroglobulin, bone resorption, antigen presentation as well as in the self-protection of cytotoxic T cell lymphocytes from their own perforins. However, changes at the cathepsin B protein level, activity and localisation are associated with several disease states, such as tumour invasion and metastasis, angiogenesis, rheumatoid arthritis, osteoarthritis, acute pancreatitis, Alzheimer's disease and apoptosis.

In cancerous diseases cathepsin B increases protein expression and activity and also alters cell localisation. In healthy cells cathepsin B is localised within perinuclear vesicles whilst in cancer cells it is translocated to the peripheral part of cytoplasm as well as to the outer surface of the cell. Furthermore, cathepsin B can be secreted from the cancer cell to the extracellular environment, thus enabling extracellular in addition to intracellular degradation of proteins of extracellular matrix *in vitro* (collagen type IV, laminin and fibronectin) and *in vivo* (collagen type IV) contributing in this way significantly to tumour invasion and metastasis.

Cathepsin B is a very attractive target for the development of new, selective and reversible cathepsin B inhibitors due to its involvement in several pathological states and also, due to the fact that there are not yet any known cathepsin B inhibitors in clinical use.

Known inhibitors of cathepsin B can be divided into two groups: endogenous protein inhibitors and exogenous synthetic inhibitors of cathepsin B.

Protein inhibitors of cysteine proteases are named cystatins and can be divided into 3 families according to their structural characteristics, physiological roles and localisation within the human body. The first family is comprised of stefins (Family I) which are 100 amino acids long intracellular inhibitors. They are not glycosylated and do not contain the signal peptide. Two main representatives of this family are stefins A and B. Cystatins (Family II) are 120 amino acid long polypeptide inhibitors which generally contain 2 disulfide bonds and the signal peptide which is responsible for their mainly extracellular localisation. Members of the cystatin family are cystatins C, D, E/M, F, G (CRES), S, SN and SA. Kininogens (Family III) are proteins with high molecular weight which contain three cystatin-like domains of which only two are capable of inhibition. Their localisation is mainly intravascular. Other families such as chagasins, clitocypins, staphostatins, calpastatins, thyropins, inhibitors of apoptosis (IAP) and individual proteins such as p41 invariant chain, equistatin, saxiphilin and p35 can also inhibit cysteine proteases. Although some of these protein inhibitors inhibit cysteine proteases and also cathepsin B in nanomolar range they are not used for the treatment of diseases associated with their altered activity. They are used solely for research purposes.

Synthetic inhibitors of cathepsin B can be in general divided into reversible and irreversible inhibitors. The majority of known cathepsin B reversible inhibitors are transition-state analogues containing electrophilic carbonyl groups and include peptidyl aldehydes, trifluoromethyl ketones, methyl ketones, α-ketoacids, α-ketoesters, α-ketoamides, α-keto-β-aldehydes and diketones. Transition-state analogues bind in the active site to form a hemithioketal adduct which is similar to tetrahedral intermediate in the enzyme reaction.

Reversible peptidyl aldehyde inhibitors inhibit both cysteine and serine proteases. Selectivity for cysteine proteases can be achieved through variation of amino acid residues in the P1 and P2 positions to match the enzyme's preferred substrates. The main disadvantage of aldehyde inhibitors is that they are "slow-binding" inhibitors due to the low concentration of free aldehyde actually present in solution, poor in vivo activity due to their low cell permeability and their susceptibility to proteolytic degradation.

Peptidyl trifluoromethyl and methyl ketones are similar to aldehyde inhibitors in inhibition of both cysteine and serine proteases, albeit they favour serine proteases.

Peptidyl α-ketoacids, α-ketoesters, α-ketoamides, α-keto-β-aldehydes in diketones are in general better inhibitors of cysteine proteases than trifluoromethyl ketones and methyl ketones and with the exception of α-keto-β-aldehydes are not better inhibitors of cysteine proteases than aldehyde inhibitors.

Peptidyl nitriles are a group of reversible inhibitors forming an isothioamide in the active site of the enzyme. They selectively inhibit cysteine proteases and are poor inhibitors of serine proteases. This selectivity is due to the higher nucleophilic character of thiolate in the active site of cysteine proteases compared to the catalytic residue of serine proteases. Furthermore, the isothioamide trigonal structure more closely resembles the substrate transition state of cysteine proteases compared to that of serine proteases. Peptidyl nitriles are poor inhibitors of cysteine proteases compared to peptidyl aldehydes.

Recently, two new classes of reversible cathepsin B inhibitors were discovered: natural biflavones which selectively inhibit cathepsin B endopeptidase activity and [2-[2-(2,4-dioxo-1,3-thiazolidin-3-yl)ethylamino]-2-oxoethyl]-2-(furan-2-carbonylamino) acetate which contains two binding sites on the enzyme and equally inhibits cathepsin B endopeptidase and exopeptidase activity. An alternative way to reversibly inhibit cathepsin B is to use specific neutralizing monoclonal antibodies. In this way, 2A2 monoclonal antibodies were prepared which specifically recognize cathepsin B and regulate its activity through inhibition of its endopeptidase and potentiation of its exopeptidase activity.

The group of irreversible cathepsin B inhibitors is comprised of: peptidyl epoxysuccinyl, aziridine, 1,2,4-thiadiazole, acyloxymethylketone, epoxide, vinyl sulfone cathepsin B inhibitors, α, β-unsaturated carbonyl derivatives, azapeptide, hydroxamate, β-lactam and organotellurium (IV) cathepsin B inhibitors.

Peptidyl epoxysuccinyl inhibitors are one of the most studied inhibitors of cathepsin B. The latter possess a reactive three-membered ring that opens when attacked by the nucleophilic thiol group in the active site of the enzyme in this way forming a thioether bond. This group of inhibitors was developed from the natural inhibitor E-64 isolated from the *Aspergillus japonicus.* E-64 is known as a potent and specific irreversible inhibitor of cysteine proteases which binds in the active site in the orientation opposite to that of their peptide substrates. His derivative CA-074, a selective inhibitor of cathepsin B, binds in the active site in the same orientation as the enzyme's substrates. Its selectivity for cathepsin B is mainly due to the presence of carboxyl group at the C-terminal part of inhibitor which forms interactions with two positively charged histidine residues in the cathepsin B occluding loop. Both inhibitors, E-64 and CA-074, inhibit cathepsin B in the nanomolar range. However, both of them have low cell-permeability due to their peptidyl structure. Esterification of the carboxylate moiety led to development of their cell permeable derivatives E-64d and CA-074Me which were 100-1000 fold less potent *in vitro but in vivo* they rapidly hydrolyzed to their active form. The main disadvantage of these inhibitors is their reactivity towards proteins other than cysteine proteases.

Aziridine inhibitors possess similar mechanism of action as epoxysuccinyl inhibitors and can be regarded as aza-analogues of epoxysuccinyl inhibitors. However, with aziridine inhibitors substituents can be placed at the nitrogen atom of the aziridine ring enabling bigger diversity in the structure of inhibitors. 1,2,4-thiadiazole cathepsin B inhibitors act as thiol trapping agents as they undergo selective ring opening with thiols forming a disulphide bond between the enzyme and inhibitor. This is not the case with amines or alcohols. Variation of substituents on C3 position influences the reactivity of ring opening whilst variation on C5 position influences recognition of cathepsin B. The special feature of 1,2,4-thiadiazole inhibitors is their inability to inhibit cathepsin B within the cell because of their fast inactivation with reduced glutathione. This enables selective inhibition of extracellular cathepsin B which is implicated in pathological processes whilst protecting the intracellular cathepsin B associated with physiological activity of the enzyme.

Acyloxymethylketone cathepsin B inhibitors also contain a peptidyl backbone responsible for specificity for cathepsin B and a reactive group forming a covalent bond with the thiol group within the active site and thus inactivating cathepsin B.

Epoxide inhibitors are relatively new inhibitors of cathepsin B and are stable at neutral and alkaline pH.

Vinyl sulfone inhibitors and α,β-unsaturated carbonyl derivatives react with cathepsin B via a Michael addition by an attack at the β-carbon by the active site cysteine, followed by a protonation of α-carbon or in the case of vinyl sulfones departure of the chlorine. Vinyl sulfones have considerable potential for use as drugs, however α,β-unsaturated carbonyl derivatives are not very potent and show little promise for future use as inhibitors for cysteine proteases.

Azapeptide inhibitors were primarily designed as serine protease inhibitors but were soon acknowledged as potent inhibitors of cysteine proteases.

Peptidyl hydroxamates were similarly as azapeptide inhibitors designed as inhibitors of serine proteases but were soon shown to be more effective as cysteine protease inhibitors. 5-Nitro-8-hydroxyquinoline, a member of 8-hydroxyquinolines, is a well known urinary tract antiseptic. Its mechanism of action is chelation of metallic ions in the active sites of metallo-enzymes in this way preventing binding of the substrates causing bacteriostatic, bactericidal and fungicidal effects. 5-nitro-8-hydroxyquinoline is an approved drug for the prevention and treatment of acute, chronic and repeating infections of the urinary tract caused by gram-positive and gram-negative bacteria and fungi, especially with children (Summary of Product Characteristics of the medicinal product 5 - NOK^{®} 50 mg oblo 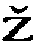 ene tablete, Lek Pharmaceuticals d.d., Ljubljana). However, the effects of 5-nitro-8-hydroxyquinoline on cathepsin B activity have not been shown yet. Furthermore, 5-nitro-8-hydroxyquinoline is not yet approved for treatment of any diseases associated with cathepsin B.

### SUMMARY OF THE INVENTION

The objective of the present invention was to provide a reversible 8-hydroxyquinoline protease inhibitor, in particular an inhibitor of cysteine proteases, particularly an inhibitor of cysteine proteases of the papain superfamily. More particularly, the present invention relates to such compounds which inhibit cysteine proteases of the cathepsin family, preferably compounds which inhibit cathepsin B. The compounds of the present invention are useful for the treatment of diseases which may be therapeutically modified by altering the activity of such proteases.

The present invention relates to the of compound having general formula **(I),** 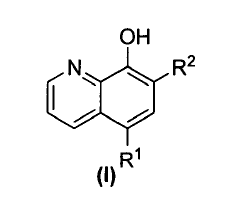 wherein: R¹ is -H, -NO₂ C₁₋₆ branched or linear alkyl optionally substituted with one or
more substituents independently selected from the group of R⁵, -NO₂, -NH₂, 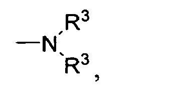 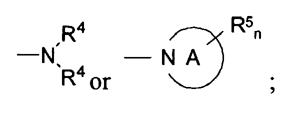 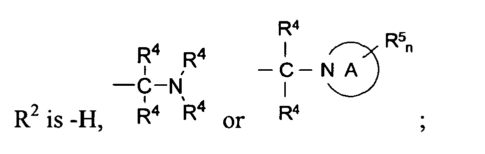 wherein A is a saturated or an unsaturated 3 to 8-membered heterocycle optionally comprising one or more further heteroatoms selected from N, O, S, and P, the heterocycle being optionally substituted with one or more substituents independently selected from R⁵, wherein n = 0 to 5;
R³ is -H, C₁₋₆ branched or linear alkyl optionally substituted with one ore more substituents independently selected from R⁵, C₃-C₆ cycloalkyl optionally substituted with one ore more substituents independently selected from R⁵;
R⁴ is -H; C₁₋₆ branched or linear alkyl optionally substituted with one ore more substituents independently selected from R⁵ and optionally comprising one or more heteroatoms selected from N, S, O, P and optionally substituted with -N(R³)_{2,} C₃-C₆ cycloalkyl optionally comprising one or more heteroatoms selected from N, S, O, P and optionally substituted with -N(R³)_{2,} aryl optionally substituted with one ore more substituents independently selected from R⁵, heteroaryl optionally substituted with one ore more substituents independently selected from R⁵;
R⁵ is -H, halo, -NH₂, -OH, -CN, -NO₂, -COOH, -CONR⁴R⁴, -SO₂NR⁴R⁴, -SO₂R⁴, -CF₃, -NHSO₂R⁴, -NHCOR⁴, C₁₋₆ branched or linear alkyl, C₁₋₆ alkoxy, -SR⁴;
wherein multiple occurrences of any of R³, R⁴ and R⁵ in the structure means that the respective residues are independently selected from the respective groups defined above;
or a pharmaceutically acceptable salt, hydrate or solvate thereof;
as an inhibitor of a cysteine protease.

The invention further relates to the use described above, wherein the cysteine protease is a cathepsin B protease.

In a preferred embodiment of the invention, the said inhibition is *ex vivo.*

In another preferred embodiment of the invention, the said inhibition is *in vivo.*

In a preferred embodiment of the invention, R¹ is selected from R⁵, -NH₂ and -NO₂.

In another preferred embodiment of the invention, R¹ is -NO₂.

In another preferred embodiment of the invention, R² is -H or

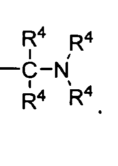

In another preferred embodiment of the invention, R² is morpholinomethyl, pyrrolidinomethyl, (4-methylpiperazin-1-yl)methyl, (4-hydroxypiperidin-1-yl)methyl, (4-methypiperidin-1-yl)methyl, (4-carboxypiperidin-1-yl)methyl, or (3-hydroxypiperidin-1-yl)methyl.

The invention further relates to compound having general formula (I), 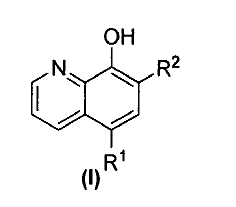 wherein: R¹ is -H, -NO₂, C₁₋₆ branched or linear alkyl optionally substituted with one or
more substituents independently selected from the group of: R⁵, -NO₂, -NH₂, 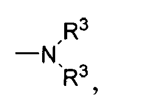 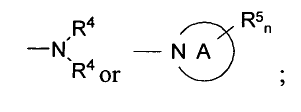 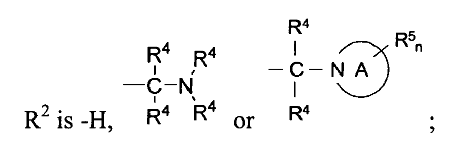 wherein A is a saturated or an unsaturated 3 to 8-membered heterocycle optionally comprising one or more further heteroatoms selected from N, O, S, or P; the heterocycle being optionally substituted with one or more substituents independently selected from R⁵ and n = 0 to 5;
R³ is -H, C₁₋₆ branched or linear alkyl optionally substituted with one ore more substituents independently selected from R⁵, C₃-C₆ cycloalkyl optionally substituted with one ore more substituents independently selected from R⁵;
R⁴ is -H; C₁₋₆ branched or linear alkyl optionally substituted with one ore more substituents independently selected from R⁵ and optionally comprising one or more heteroatoms selected from N, S, O, P and optionally substituted with -N(R³)₂; C₃-C₆ cycloalkyl optionally comprising one or more heteroatoms selected from N, S, O, P and optionally substituted with -N(R³)₂; aryl optionally substituted with one ore more substituents independently selected from R⁵; heteroaryl optionally substituted with one ore more substituents independently selected from R⁵;
R⁵ is -H, halo, -NH₂, -OH, -CN, -NO₂, -COOH, -CONR⁴R⁴, -SO₂NR⁴R⁴, -SO₂R⁴, -CF₃, -NHSO₂R⁴, -NHCOR⁴, C₁₋₆ branched or linear alkyl, C₁₋₆ alkoxy, SR⁴;
wherein multiple occurrences of any of R³, R⁴ and R⁵ in a single structure means that the respective residues are independently selected from the members of the respective groups;
or a pharmaceutically acceptable salt, hydrate or solvate thereof;
for use as a medicament.

The invention further relates to methods of treating a subject in need with a substance of the invention. The invention further relates to methods of manufacturing a pharmaceutical composition useful in the treatment of medical indications of the invention, said pharmaceutical composition comprising a substance of the present invention.

In another preferred embodiment of the invention, said medicament is useful in the treatment of a disease in a patient, wherein said disease is associated with an abnormal activity of a cysteine protease in said patient, preferably with an abnormal activity of cathepsin B protease in said patient.

In another preferred embodiment of the invention, said disease is cancer, rheumatoid arthritis, osteoarthritis, osteoporosis, pancreatitis, an immune disease, a neurodegenerative disease or Alzheimer's disease. In a more preferred embodiment of the invention, said disease is osteoporosis, pancreatitis, an immune disease, a neurodegenerative disease or Alzheimer's disease.

In another preferred embodiment of the invention, said patient is a human or a mammal.

The invention also relates to compounds according to the above definition, wherein R¹ is R⁵, -NH₂ or -NO₂.

In another preferred embodiment of the invention, R¹ is -NO₂.

In another preferred embodiment of the invention, R² is -H or

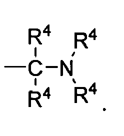

In another preferred embodiment of the invention, R² is morpholinomethyl, pyrrolidinomethyl, (4-methylpiperazin-1-yl)methyl, (4-hydroxypiperidin-1-yl)methyl, (4-methypiperidin-1-yl)methyl, (4-carboxypiperidin-1-yl)methyl, or (3-hydroxypiperidin-1-yl)methyl.

The invention further relates to precursor compounds, i.e., compounds which are converted to the above defined compounds *in vivo.*

### BRIEF DESCRIPTION OF THE DRAWINGS

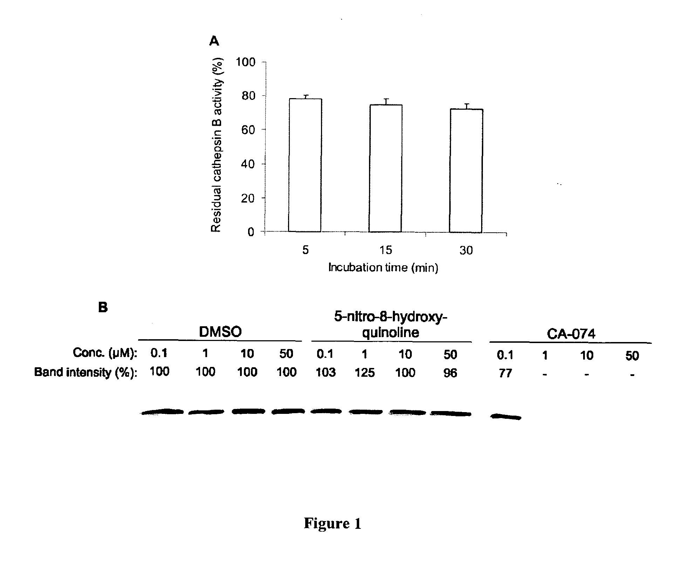

**Figure 1****.** Determination of reversibility of cathepsin B inhibition by 5-nitro-8-hydroxyquinoline using enzyme kinetics (A) and active site probe DCG-04 (B). Numbers above individual bands represent percentage of intensity of DMSO control, respectively.

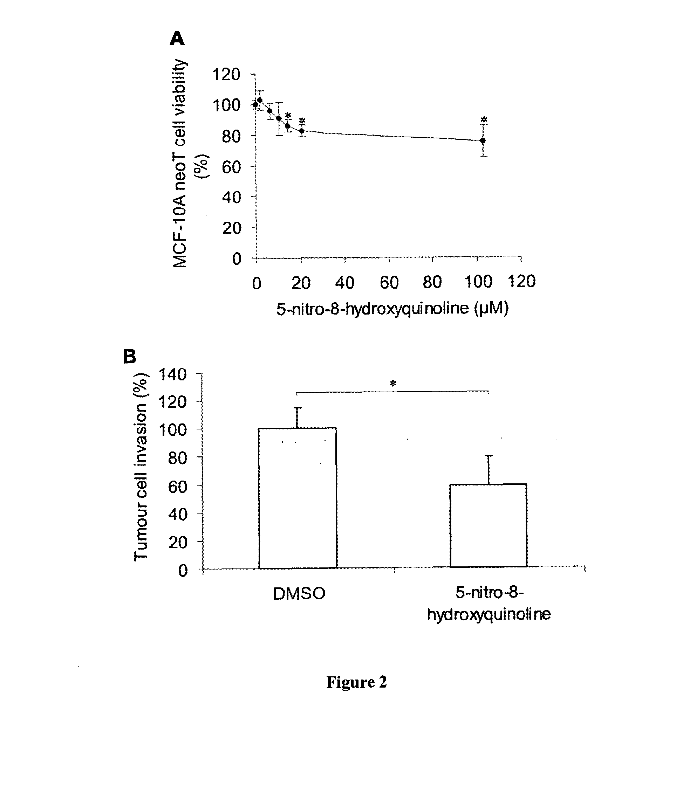

**Figure 2.**(A) Effect of 5-nitro-8-hydroxyquinoline on MCF-10A neoT cell viability as evaluated using the MTS assay. Data are presented as the mean ± SD of quadruplicate wells. Significant cytotoxicity is marked with asterisk (p ≤ 0.05). (B) Effect of 5-nitro-8-hydroxyquinoline at 10 µM on tumour cell invasion as assessed with Millicell Matrigel invasion assay. Data are presented as the mean ± SD of triplicate wells. Significant inhibition of invasion is marked with asterisk (p ≤ 0.05).

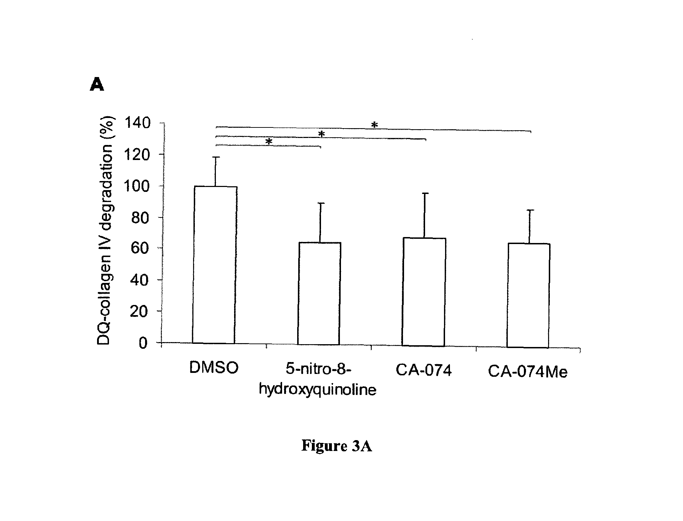

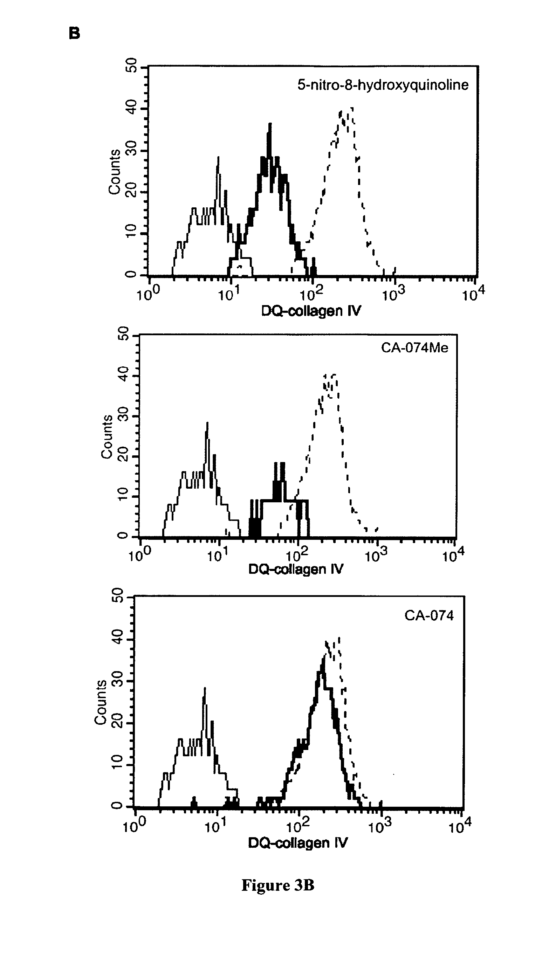

**Figure 3.** (A) 5-nitro-8-hydroxyquinoline significantly inhibits extracellular DQ-collagen IV degradation (35.3 %) as well as CA-074 (31.1 %) and CA-074Me (33.8 %) (p ≤ 0.05). (B) 5-nitro-8-hydroxyquinoline significantly inhibits intracellular DQ-collagen IV degradation as measured by flow cytometry (p ≤ 0.05). Inhibition of DQ-collagen IV degradation can be seen as a shift in fluorescence intensity (thick black line) as compared to MCF-10A neoT cells grown in the presence of DMSO, used as a control (thin black line). As control cells were grown in the absence of DQ-collagen IV (dashed black line). Permeable cathepsin B inhibitor CA-074Me was used as a positive control and also significantly inhibited intracellular degradation of DQ-collagen IV, whereas impermeable cathepsin B inhibitor CA-074 did not.

### DETAILED DESCRIPTION OF THE INVENTION

"Alkyl", as used hereinbelow, shall be understood to relate to a branched or linear alkyl group with 1 to 20, preferably 1 to 10, most preferred 1 to 5 or 1 to 3 carbon atoms. Linear alkyl groups are preferred.

A "heterocycle", as used hereinbelow, shall be understood to relate to cyclic moieties comprising 2 to 7 carbon atoms in addition to at least one heteroatom in the cycle. Preferred heterocycles comprise 2 to 5, or 2 to 4 carbon atoms. Preferred heteroatoms are N, S, O, P. Preferred heterocycles of the invention comprise a single heteroatom.

"Branched alkyl", as used hereinbelow, shall be understood to relate to a branched alkyl group with 1 to 20, preferably 1 to 10, most preferred 1 to 5 or 1 to 3 carbon atoms.

"Aryl", as used hereinbelow, shall be understood to relate to any functional group or substituent derived from a simple aromatic ring, may it be phenyl, thiophene, indolyl, etc (according to IUPAC nomenclature). Preferred aryl groups are phenyl, C₆H₅; tolyl, CH₃C₆H₄; or xylyl, (CH₃)₂C₆H₃. Preferred aryl groups of the invention comprise 3 to 6 membered carbon cycles, most preferred 6 membered carbon cycles. Aryl groups of the invention may comprise a single aromatic cycle, but may also comprise two, or three, or more conjugated aromatic cycles. A single aromatic cycle is preferred.

"Heteroaryl" shall be understood to relate to aryl comprising a heteroatom in at least one of the aromatic cycles.

Main advantages of 8-hydroxyquinoline compounds of the invention over the established inhibitors of cathepsin B are as follows:
Vast majority of known cathepsin B inhibitors are irreversible inhibitors. The latter possess questionable *in vivo* characteristics, selectivity in particular. As a consequence, they tend to cause more side effects than reversible inhibitors when used in therapy and are therefore less suitable for the use in treatment of diseases. 8-hydroxyquinolines, in particular 5-nitro-8-hydroxyquinoline, are reversible cathepsin B inhibitors and have thus greater potential for the use in treatment of diseases in which cathepsin B is implicated compared to irreversible inhibitors of cathepsin B.

5-nitro-8-hydroxyquinoline in comparison with other known reversible inhibitors of cathepsin B is more selective towards enzyme's endopeptidase activity. Furthermore, it has a lower molecular weight meaning that it has also greater potential for modification and preparation of analogues.

5-nitro-8-hydroxyquinoline is a low-molecular weight inhibitor and does not possess the peptidyl structure as the majority of known cathepsin B inhibitors. Peptidyl derivatives have lower bioavailability and are susceptible to proteolytic degradation. For example, irreversible cathepsin B inhibitor CA-074 is a potent peptidyl inhibitor of cathepsin B *in vitro,* however due to its charged nature it has low cell permeability and therefore cannot inhibit the intracellular cathepsin B. This problem was solved with preparation of the methyl ester CA-074Me which indeed could cross the cell membrane but at the same time had lower selectivity since it also inhibited cathepsin L. 5-nitro-8-hydroxyquinoline does not have the peptidyl structure and has therefore better bioavailability than peptidyl inhibitors (it efficiently inhibits both intracellular and extracellular cathepsin B) and is also resistant to proteolytic degradation.

5-nitro-8-hydroxyquinoline more effectively inhibits cathepsin B endopeptidase activity compared to its exopeptidase activity. The majority of known cathepsin B inhibitors accomplish their selectivity for cathepsin B through interaction with two histidine residues located in the occluding loop. However, the occluding loop is essential for the exopeptidase activity of cathepsin B which is usually associated with physiological activity of the enzyme. This is the reason why the majority of known cathepsin B inhibitors primarily inhibit its exopeptidase and are less effective at inhibition of its endopeptidase activity which is essential for enzyme's role in tumour invasion and metastasis. So far only two such cathepsin B inhibitors are known: 2A2 monoclonal antibody and biflavone inhibitors.

The present invention relates to the use of compound as described in the above "Summary of the invention" as a medicament.

The present invention includes all hydrates, solvates, complexes, polymorphs and prodrugs of the compounds corresponding to Formula (**I**). Prodrugs are any covalently bonded compounds which release the active parent drug according to Formula (**I**) *in vivo.*

All forms of isomers resulting from the presence of a chiral center in the inventive compounds, including enantiomers and diastereomers, are intended to be covered herein. The inventive compounds may be used as a racemic mixture, an enantiomerically enriched mixture, or the racemic mixture may be separated using well-known techniques and an individual enantiomer may be used alone.

In the event that the present compound may exist in tautomeric forms, each tautomeric form is considered as being included within this invention whether existing in equilibrium or predominantly in one form.

### Utility of present compounds

The present invention provides a pharmaceutical composition which consists of a compound according to Formula (**I**) and a pharmaceutically acceptable carrier, excipient or diluent. Furthermore the compound according to Formula (**I**) can also be used in the preparation of a medicament. Pharmaceutical preparations containing the compound from Formula (**I**) may be formulated as solutions or lyophilized powders for parenteral administration. In the latter case, powders may be reconstituted by addition of a suitable diluent or other pharmaceutically acceptable carrier prior to use. The liquid preparation may be buffered, isotonic, aqueous solution. Examples of suitable diluents are normal isotonic saline, standard 5 % dextrose in water, or buffered sodium or ammonium acetate solution. Such formulation is especially suitable for parenteral administration, but may be also used for oral administration or contained in a metered dose inhaler or nebulizer for insufflation. It may be desirable to add excipients such as polyvinylpyrrolidone, gelatin, hydroxy cellulose, acacia, polyethylene glycol, mannitol, sodium chloride, or sodium citrate.

Alternatively, the compounds described in the Formula (**I**) may be encapsulated, tableted, or prepared in an emulsion or syrup for oral administration. Pharmaceutically acceptable solid or liquid carriers may be added to enhance or stabilize the composition, or to facilitate preparation of the composition. Solid carriers include starch, lactose, calcium sulfate dihydrate, terra alba, magnesium stearate or stearic acid, talc, pectin, acacia, agar or gelatin. Liquid carriers include syrup, peanut oil, olive oil, saline and water. The carrier may also include a sustained release material such as glyceryl monostearate or glyceryl distearate, alone or with a wax. The amount of solid carrier may vary, but will preferably be between about 20 mg to about 1 g per dosage unit. The pharmaceutical preparations are made following the conventional pharmaceutical techniques, such as milling, mixing, granulating, and compressing when necessary for tablet forms; or milling, mixing and filling for hard gelatin capsule forms. When a liquid carrier is used, the preparation will be in the form of syrup, elixir, emulsion or an aqueous or a non-aqueous suspension. Such a liquid formulation may be administered directly or filled in a soft gelatin capsule.

For rectal administration, the compound of this invention may also be combined with excipients, such as cocoa butter, glycerin, gelatin or polyethylene glycols and moulded into a suppository.

Compounds according to Formula (I) are useful as protease inhibitors, particularly as inhibitors of cysteine proteases, more particular as inhibitors of cysteine proteases of the papain superfamily, yet more particularly as inhibitors of cysteine proteases of cathepsin family, most particularly as inhibitors of cathepsin B. The present invention also provides useful compositions and formulations of said compounds.

The present compounds are useful for treating diseases in which cysteine proteases are implicated and especially diseases in which cathepsin B is implicated, such as cancer, rheumatoid arthritis, osteoarthritis, osteoporosis, pancreatitis, immune and neurodegenerative diseases, e.g. Alzheimer's disease.

The present invention also provides methods of treatment of diseases caused by pathological levels of proteases, particularly cysteine proteases, more particularly cysteine proteases of the papain superfamily, even more particularly cysteine proteases of the cathepsin family, which methods comprise administering to an animal, particularly a mammal, most particularly a human in need thereof the compounds of the present invention. The present invention especially provides methods of treatment of diseases caused by pathological levels of cathepsin B, which methods comprise administering to an animal, particularly a mammal, most particularly a human in need thereof, an inhibitor of cathepsin B, including the compounds of the present invention. The present invention particularly provides methods for treating diseases in which cysteine proteases are implicated and especially diseases in which cathepsin B is implicated, such as cancer, rheumatoid arthritis, osteoarthritis, osteoporosis, pancreatitis, immune and neurodegenerative diseases, e.g. Alzheimer's disease.

This invention further provides a method for treating cancer, rheumatoid arthritis, osteoarthritis, osteoporosis, pancreatitis, immune and neurodegenerative diseases, e.g. Alzheimer's disease, which comprises of internal administration to a patient of an effective amount of the compounds with Formula (I), alone or in combination with other drugs used for the treatment of the respective diseases.

In accordance with this invention, an effective amount of compound corresponding to Formula (**I**) is administered to inhibit the protease implicated in a particular condition or a disease. The dosage amount can be further modified according to the type of administration of the compound. For example, for acute therapy, parenteral administration of the compound corresponding to Formula (**I**) is preferred. An intravenous infusion of the compound in 5 % dextrose in water or normal saline, or similar formulation with suitable excipients is most effective, although an intramuscular bolus injection is also useful. Typically, the parenteral dose will be about 0.01 to about 100 mg/kg, in a manner to maintain the concentration of drug in the plasma at a concentration effective to inhibit cathepsin B. The compound is administered one to four times daily at a level to achieve a total daily dose of about 0.4 to about 400 mg/kg/day. The precise amount of the inventive compound which is therapeutically effective, and the route by which such compound is best administered, is readily determined by one of the ordinary skill in the art by comparing blood levels of the agent to the concentration required to have a therapeutic effect.

Prodrugs of the compound corresponding to Formula (**I**) may be prepared by any suitable method. Where the prodrug moiety is a ketone functionality, specifically ketals and/or hemiacetals, the conversion may be effected in accordance with conventional methods.

The compounds corresponding to Formula (**I**) may be administered orally to the patient, in a manner such that the concentration of the drug is sufficient to achieve any therapeutic indication as disclosed herein. Typically a pharmaceutical composition containing the compound is administered at an oral dose of between about 0.1 to about 50 mg/kg in a manner consistent with the condition of the patient.

### Medical indications

The present invention also relates to the compounds of the invention for use as a medicament in cancer, rheumatoid arthritis, osteoarthritis, osteoporosis, pancreatitis, an immune disease, a neurodegenerative disease or Alzheimer's disease.

### Cancer

Increased levels of cathepsin B protein and activity are found in tumour tissues and have been suggested as prognostic markers in patients with breast, lung, colon, and ovarian carcinomas, as well as gliomas and melanomas. The localization of cathepsin B in transformed and tumour cells has been shown to change from the perinuclear vesicles, as found in normal cells, to the peripheral cytoplasmic regions. Moreover, in tumour cells, cathepsin B can be secreted into the extracellular environment or be associated with the cell surface. The secreted cathepsin B can activate other proteases acting downstream in the catalytic cascade or directly degrade the extracellular matrix (ECM) proteins. Cathepsin B contributes to both intracellular and pericellular degradation of ECM proteins, both *in vitro* (type IV collagen, laminin, fibronectin) and *in vivo* (type IV collagen), implicating its role in malignant disease by facilitating tumour invasion and metastasis. Inhibitors of cathepsin B of the present invention are thus useful as medicaments in the treatment of cancer.

### Rheumatoid arthritis

It was shown recently that cathepsin B activity was significantly increased in synovial fluid of patients with rheumatoid disease and that the enzyme from the synovial fluid could degrade collagen. This activity was successfully inhibited with a specific cathepsin B inhibitor, CA-074 suggesting that cathepsin B may participate in joint destruction in rheumatoid arthritis. Inhibitors of cathepsin B of the present invention are thus useful as medicaments in the treatment of rheumatoid arthritis.

### Osteoarthritis

Increased cathepsin B mRNA and protein levels in a cell-based osteoarthritis model and increased levels of cathepsin B in human osteoarthritis cartilage suggest a possible role for cathepsin B in osteoarthritis. It was proposed that cathepsin B promotes osteoarthritis through degradation of collagen and aggrecan and through destruction of TIMP-1 and TIMP-2 in this way upregulating the activity of matrix metalloproteinases. Inhibitors of cathepsin B of the present invention are thus useful as medicaments in the treatment of osteoarthritis.

### Osteoporosis

Cathepsin B was shown previously to participate in bone *resorption in vitro and in vivo.* It was suggested that the enzyme was responsible for intracellular activation of bone resorbing enzymes (matrix metalloproteinases, procollagenase, prostromelysin and prourokinase-type plasminogen activator) which afterwards participate in collagen degradation. Therefore, inhibition of cathepsin B could be valuable in treatment of diseases with increased bone resorption, e.g. osteoporosis. Inhibitors of cathepsin B of the present invention are thus useful as medicaments in the treatment of osteoporosis.

### Pancreatitis

Cathepsin B plays an essential role in pancreatitis through trypsinogen activation resulting in the active enzyme form and the subsequent autodigestion of the gland. It was shown previously that trypsin activity was reduced 80 % and that the extent of acinar tissue necrosis was 50 % lower in cathepsin B-deficient mice with induced pancreatitis compared to control. Furthermore, it was also shown that cathepsin B inhibition prevents trypsinogen activation and reduces pancreatitis severity. Inhibitors of cathepsin B of the present invention are thus useful as medicaments in the treatment of pancreatitis.

### Immune diseases

Cathepsin B is involved in the trafficking of TNF-α-containing vesicles to the plasma membrane in macrophages. It is known that TNF- α participates in severe acute and inflammatory diseases. Therefore, cathepsin B inhibitors could be used also as antiinflammatory therapeutics. Inhibitors of cathepsin B of the present invention are thus useful as medicaments in the treatment of immune diseases.

### Neurodegenerative diseases

Cathepsin B was shown to participate in neurodegenerative diseases, for example Alzheimer's disease (see below). Furthermore, altered cathepsin B activity was associated with amyotrophic lateral sclerosis and inflammatory neurologic diseases, such as multiple sclerosis, Guillain-Barré syndrome and chronic inflammatory demyelinating polyneuropathy. Inhibitors of cathepsin B of the present invention are thus useful as medicaments in the treatment of neurodegenerative diseases.

### Alzheimer's disease

Cathepsin B was recently discovered to participate in β-secretase cleavage of amyloid precursor protein in this way producing neurotoxic β-amyloid peptides in the brain. The latter is a major factor involved in the development and pathogenesis of Alzheimer's disease, which results in loss of memory and cognitive functions. Furthermore, it was shown that cathepsin B inhibition leads to reduced levels of neurotoxic β-amyloid peptides and improves memory and cognitive functions. Inhibitors of cathepsin B of the present invention are thus useful as medicaments in the treatment of Alzheimer's disease.

### EXAMPLES

### Definitions

Standard abbreviations and symbols from biochemistry and chemistry field are used to denote the compounds and reagents presented in this invention. In particular, DMSO means dimethyl sulfoxide, Ki means constant of inhibition, IC₅₀ means concentration of inhibitor which inhibits 50 % of enzyme activity, PBS means phosphate buffered saline.

### General synthetic procedure

To a stirred and heated (55 °C) solution of 5-nitro-8-hydroxyquinoline (1) (5 mmol, 950.8 mg) in pyridine (20 mL) an aqueous solution of formaldehyde (≥36.5 %, 1 mL) and a desired secondary amine (5 mmol) are added. After heating the reaction mixture at 55 °C for 90 min, it is cooled to room temperature, the formed product filtrated and washed with cold EtOH (2 x 20 mL). The compounds are subsequently crystallized from proper solvent (Movrin M, Maysinger D, Marok E: Biologically Active Mannich Bases Derived from Nitroxoline. Pharmazie 1980, 35: 458-60.).

### Examples:

### 7-(Morpholinomethyl)-5-nitro-8-hydroxyquinoline

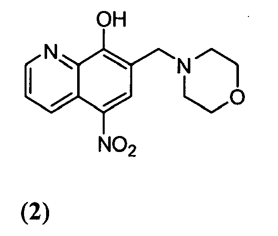

The compound is crystallized from CHCl₃.

Description: slightly hygroscopic yellow crystals

Yield: 75%

ESI-MS (M+H)⁺: m/z = 290

HRMS (m/z = 290) for C₁₄H₁₆N₃O₄: calculated 290.1141, found 290.1155.

¹H-NMR (300 MHz, CF₃COOD): δ (ppm) = 3.50-3.64 (m, 2H, morpholine-C*H*₂), 3.67-3.79 (m, 2H, morpholine-C*H*₂), 4.06 (t, *J* = 12.5 Hz, 2H, morpholine-C*H*₂), 4.26-4.38 (m, 2H, morpholine-C*H*₂), 4.85 (s, 2H, -C*H*₂-N), 8.31-8.41 (m, 1H, Ar-*H*), 9.01 (d, *J* = 1.2 Hz, 1H, Ar-*H*), 9.21 *(d, J = 5.5 Hz,* 1H, Ar-*H*, 10.17 *(d, J = 9.0 Hz,* 1H, Ar-*H*).

Elemental analysis:
calculated (%) for C₁₄H₁₅N₃O₃×0.22 H₂O: C, 57.34; H, 5.31; N, 14.33
found (%): C, 57.34; H, 5.28; N, 14.27.

### 7-(Pyrrolidinomethyl)-5-nitro-8-hydroxyquinoline

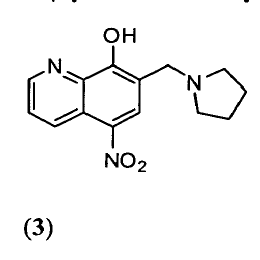

The compound is crystallized from CH₂Cl₂.

Description: yellow crystals

Yield: 82%

ESI-MS (M+H)⁺: m/z = 274

HRMS (m/z = 274) for C₁₄H₁₆N₃O₃: calculated 274.1192, found 274.1180.

¹H-NMR (300 MHz, CF₃COOD): δ (ppm) = 1.91-2.23 (m, 4H, 2 × pyrrolidine-C*H*₂), 3.11-3.29 (m, 2H, pyrrolidine-C*H*₂), 3.54-3.71 (m, 2H, pyrrolidine-C*H*₂), 4.64 (s, 2H, - C*H*₂-N), 8.14-8.25 (m, 1H, Ar-*H*), 8.81 (s, 1H, Ar-H), 9.06 (d, *J* = 5.5 Hz, 1H, Ar-*H*), 9.98 (d, *J* = 9.0 Hz, 1H, Ar-*H*).

Elemental analysis:
calculated (%) for C₁₄H₁₅N₃O₃: C, 61.53; H, 5.53; N, 15.38
found (%): C, 61.35; H, 5.38; N, 15.18.

### 7-((4-Methylpiperazin-1-yl)methyl)-5-nitro-8-hydroxyquinoline

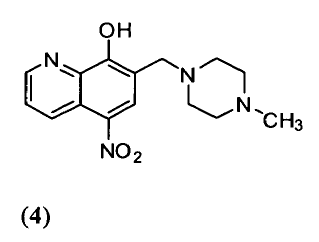

The compound is crystallized from CH₂Cl₂.

Description: yellow crystals

Yield: 65 %

ESI-MS (M+H)⁺: m/z = 303

HRMS (m/z = 303) for C₁₅H₁₉N₄O₃: calculated 303.1457, found 303.1460.

¹H-NMR (300 MHz, CF₃COOD): δ (ppm) = 3.09 (s, 3H, -C*H*₃), 3.62-3.80 (m, 2H, piperazine-C*H*₂), 3.87-4.12 (m, 6H, 3 × piperazine-C*H*₂), 4.89 (s, 2H, -C*H*₂-N), 8.24-8.35 (m, 1H, Ar-*H*), 8.95 (s, 1H, Ar-*H*), 9.13 (d, *J* = 5.5 Hz, 1H, Ar-*H*), 10.12 (d, *J* = 9.0 Hz, 1H, Ar-*H*).

Elemental analysis:
calculated (%) for C₁₅H₁₈N₃O₄: C, 59.59; H, 6.00; N, 18.53
found (%): C, 59.90; H, 5.86; N, 18.85.

### 7-((4-Hydroxypiperidin-1-yl)methyl)-5-nitro-8-hydroxyquinoline

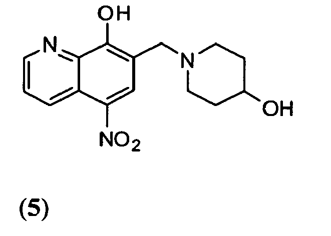

The compound is crystallized from CH₂Cl₂ and MeOH (9/1).

Description: slightly hygroscopic yellow crystals

Yield: 76%

ESI-MS (M+H)⁺: m/z = 304

HRMS (m/z = 304) for C₁₅H₁₈N₃O₄: calculated 304.1297, found 304.1303.

¹H-NMR (300 MHz, CF₃COOD): δ (ppm) = 1.93-2.42 (m, 4H, 2 × piperidine-C*H*₂), 3.24-3.84 (m, 4H, 2 × piperidine-C*H₂*), 4.13-4.47 (m, 1H, piperidine-*CH*), 4.77 (s, 2H, -C*H*₂-N), 8.29-8.39 (m, 1H, Ar-H), 8.95 (s, 1H, Ar-*H*), 9.19 (d, J = 5.0 Hz, 1H, Ar-*H*), 10.13 (d, *J* = 9.0 Hz, 1H, Ar-*H*).

Elemental analysis:
calculated (%) for C₁₅H₁₇N₃O₄×0.5 H₂O: C, 57.68; H, 5.81; N, 13.45
found (%): C, 57.72; H, 6.05; N, 13.31.

### 7-((4-Methypiperidin-1-yl)methyl)-5-nitro-8-hydroxyquinoline

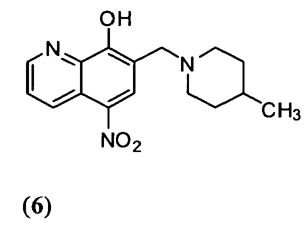

The compound is crystallized from CH₂Cl₂.

Description: yellow crystals

Yield: 83%

ESI-MS (M+H)⁺: m/z = 302

HRMS (m/z = 302) for C₁₆H₂₀N₃O₃: calculated 302.1505, found 302.1510.

¹H-NMR (300 MHz, CF₃COOD): δ (ppm) = 0.92 (d, *J=* 6.5 Hz, 3H, -C*H₃*), 1.34-1.54 (m, 2H, piperidine-C*H*₂), 1.58-1.79 (m, 1H, piperidine-C*H*), 1.83-2.00 (m, 2H, piperidine-C*H*₂), 3.13 (t, *J* = 12.0 Hz, 2H, piperidine-C*H*₂), 3.55-3.67 (m, 2H, piperidine-C*H*₂),4.64 (s, 2H, -C*H*₂-N), 8.25-8.34 (m, 1H, Ar-*H*), 8.87 (s, 1H, Ar-*H*), 9.14 (d, *J*= 5.5 Hz, 1H, Ar-*H*), 10.07 (d, *J* = 9.0 Hz, 1H, Ar-*H*).

Elemental analysis:
calculated (%) for C₁₆H₁₉N₃O₃: C, 63.77; H, 6.36; N, 13.94
found (%): C, 63.95; H, 6.31; N, 13.99.

### 7-((4-Carboxypiperidin-1-yl)methyl)-5-nitro-8-hydroxyquinoline

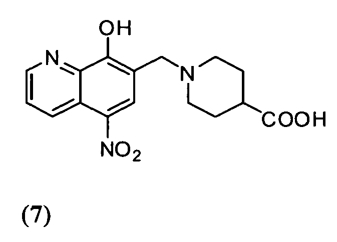

The compound is crystallized from CH₂Cl₂.

Description: slightly hygroscopic yellow crystals

Yield: 78%

ESI-MS (M+H)⁺: m/z = 332

HRMS (m/z = 332) for C₁₆H₁₈N₃O₅: calculated 332.1246, found 332.1252.

¹H-NMR (300 MHz, CF₃COOD): δ (ppm) = 2.00-2.31 (m, 2H, piperidine-C*H*₂), 2.34-2.54 (m, 2H, piperidine-C*H*₂), 2.77-3.08 (m, 1H, piperidine-C*H*), 3.25-3.55 (m, 2H, piperidine-C*H*₂), 3.62-3.90 (m, 2H, piperidine-C*H*₂), 4.78 (s, 2H, -C*H*₂-N), 8.31-8.41 (m, 1H, Ar-*H*), 8.96 (s, 1H, Ar-H), 9.21 (d, *J =* 5.5 Hz, 1H, Ar-H), 10.16 (d, *J =* 9.0 Hz, 1H, Ar-*H*).

Elemental analysis:
calculated (%) for C₁₆H₁₇N₃O₅×0.25 H₂O: C, 57.22; H, 5.25; N, 12.51
found (%): C, 57.19; H, 5.17; N, 12.35.

### 7-((3-Hydroxypiperidin-1-yl)methyl)-5-nitro-8-hydroxyquinoline, racemate

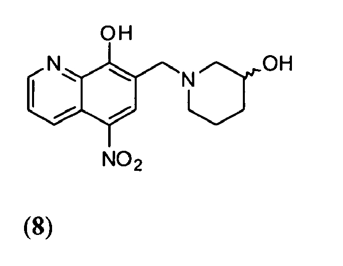

The compound is crystallized from CH₂Cl₂.

Description: yellow crystals

Yield: 63%

ESI-MS (M+H)⁺: m/z = 304

HRMS (m/z = 304) for C₁₅H₁₈N₃O₄: calculated 304.1297, found 304.1505.

¹H-NMR (300 MHz, CF₃COOD): δ (ppm) = 1.81-2.04 (m, 2H, piperidine-C*H*₂), 2.08-2.44 (m, 2H, piperidine-C*H*₂), 3.25-3.54 (m, 2H, piperidine-C*H*₂), 3.63-3.84 (m, 2H, piperidine-C*H*₂), 4.37-4.60 (m, 1H, piperidine-C*H*), 4.68-4.90 (m, 2H, -C*H*₂-N), 8.31-8.44 (m, 1H, Ar-*H*), 8.98-9.09 (m, 1H, Ar-*H*), 9.18-9.29 (m, 1H, Ar-*H*), 10.19 (d, *J* = 9.0 Hz, 1H, Ar-*H*).

### Elemental analysis:

calculated (%) for C₁₅H₁₇N₃O₄: C, 59.40; H, 5.65; N, 13.85
found (%): C, 59.24; H, 5.48; N, 13.58.

### Biological assays

The compounds of this invention were evaluated for their potency using several different biological assays.

### Effect of 8-hydroxyquinolines on cathepsin B enzymatic activity

The effect of 8-hydroxyquinolines on cathepsin B enzymatic activity was assessed using enzyme kinetics. First, the effect of 8-hydroxyquinolines on cathepsin B endopeptidase activity was assessed using the cathepsin B specific endopeptidase substrate Z-RR-AMC. 5 µl of the Z-RR-AMC (final concentration 60, 180 and 360 µM) and 5 µl of respective compound at increasing concentrations were added to wells of a black microplate. 90 µl of human recombinant cathepsin B was then added to a final concentration of 0.4 nM in activation buffer (100 mM phosphate buffer, pH 6.0 with 0.1 % PEG, 5 mM DTT and 1.5 mM EDTA). All assay mixtures contained a constant amount of DMSO, i.e. 5 %. Formation of fluorescent degradation product was continuously monitored at 380 nm ± 20 nm excitation and 460 m ± 10 nm emission wavelengths on Tecan Safire^{2™} at 37°C. Mechanism and the constant of inhibition (Ki) of cathepsin B endopeptidase activity were determined using the graphical method according to Cornish-Bowden (Cornish-Bowden A: A Simple Graphical Method for Determining the Inhibition Constants of Mixed, Uncompetitive and Non-Competitive Inhibitors. Biochem J 1974, 137:143-4.) and using the SigmaPlot^{®} 11, Enzyme Kinetics Module™ 1.3.

**Table I. Results of testing the compounds according to Formula (I) on cathepsin B endopeptidase activity.**

| Compound number | Compound structure | Ki (µM) |
|---|---|---|
| 1 | 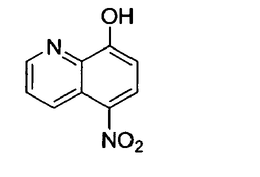 | 37.7 ± 3.5 |
| 2 | 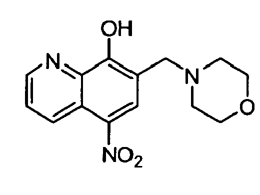 | 118.8 ± 4.2 |
| 3 | 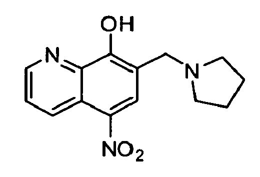 | 117.8 ± 3.1 |
| 4 | 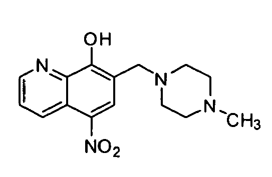 | 75.6 ± 4.0 |
| 5 | 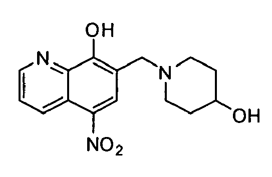 | 129.7 ± 3.4 |
| 6 | 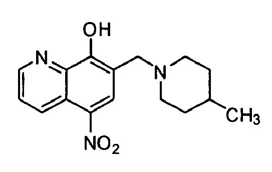 | 129.8 ± 3.5 |
| 7 | 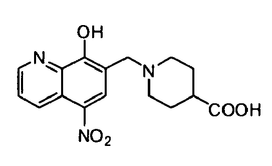 | 89.0 ± 3.3 |
| 8 | 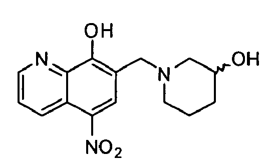 | 109.8 ± 3.1 |

One skilled in the art would consider any compound with a Ki less than 50 µM to be a potential lead compound. Of the tested 8-hydroxyquinolines the compound 5-nitro-8-hydroxyquinoline (1) proved to be the most potent inhibitor of cathepsin B with a Ki of 37.7 ± 3.5 µM (uncompetitive inhibition). Therefore, this compound was chosen for further characterisation as the representative of 8-hydroxyquinolines.

The effect of 5-nitro-8-hydroxyquinoline on cathepsin B exopeptidase activity was assessed with an exopeptidase substrate Abz-GIVRAK(Dnp)-OH. 5 µl of Abz-GIVRAK(Dnp)-OH (final concentration 1, 3 and 6 µM) and 5 µl of 5-nitro-8-hydroxyquinoline at increasing concentrations were added to wells of a black microplate. 90 µl of human recombinant cathepsin B was then added to a final concentration of 0.25 nM in activation buffer (60 mM acetate buffer, pH 5.0 with 0.1 % PEG, 5 mM DTT and 1.5 mM EDTA). All assay mixtures contained a constant amount of DMSO, i.e. 5 %.

Formation of fluorescent degradation product was continuously monitored at 320 nm ± 20 nm excitation and 420 nm ± 10 nm emission wavelengths on Tecan Safire^{2™} at 37°C. Mechanism and the constant of inhibition (Ki) of cathepsin B exopeptidase activity were determined using the graphical method according to Cornish-Bowden (Cornish-Bowden A: A Simple Graphical Method for Determining the Inhibition Constants of Mixed, Uncompetitive and Non-Competitive Inhibitors. Biochem J 1974, 137:143-4.) and using the SigmaPlot^{®} 11, Enzyme Kinetics Module™ 1.3.

5-nitro-8-hydroxyquinoline is a non-competitive inhibitor of cathepsin B exopeptidase activity with a Ki of 219.7±13.3 µM. Therefore, 5-nitro-8-hydroxyquinoline is a selective inhibitor of cathepsin B endopeptidase activity, since it inhibits its endopeptidase activity 5.8-times more potently than it does its exopeptidase activity.

Selectivity of 5-nitro-8-hydroxyquinoline for cathepsin B was assessed by determination of Ki for cathepsins H and L. Ki was determined same as before: for cathepsin L (final concentration 0.15 nM) substrate Z-FR-AMC was used at concentrations 0.5, 1 in 4 µM and for cathepsin H (final concentration 3 nM) substrate R-AMC was used at concentrations 20, 60 in 160 µM. Activation buffer used with cathepsin L was 100 mM acetate buffer, pH 5.5 with 0.1 % PEG, 5 mM DTT and 1.5 mM EDTA and with cathepsin H was 100 mM phosphate buffer, pH 6.8 with 0.1 % PEG, 5 mM DTT and 1.5 mM EDTA.

**Table II. Ki values for 5-nitro-8-hydroxyquinoline determined against cathepsins B, L and H.**

| | Cathepsin B | Cathepsin L | Cathepsin H |
|---|---|---|---|
| Mechanism of inhibition | uncompetitive | competitive | mixed |
| Ki (µM) | - | 175.3 ± 17.1 µM | 220.8 ± 2.1 µM |
| Ki' (µM) | 37.7 ± 3.5 µM | - | 301.2 ± 10.3 µM |

As evident from the Table II 5-nitro-8-hydroxyquinoline is a selective cathepsin B inhibitor.

### Reversibility of cathepsin B inhibition with 5-nitro-8-hydroxyquinoline

Reversibility of cathepsin B inhibition with 5-nitro-8-hydroxyquinoline was determined with three independent experiments. First, we used enzyme kinetics. Cathepsin B at 100-fold its final assay concentration (10 nM) and 5-nitro-8-hydroxyquinoline at 10-fold its IC₅₀ were incubated for 5, 15 and 30 min at room temperature at 2 µl. The enzyme-inhibitor complex was then diluted 100-fold with 5 µM Z-RR-AMC to a final volume of 200 µl, initiating the reaction. Before the dilution of the enzyme-inhibitor complex the enzyme's activity is inhibited by 90 %. In the case of a reversible inhibitor after dilution this percentage is reduced to 9 %. In the case of an irreversible inhibitor the percentage stays unchanged (90 %).

After 5 min incubation of cathepsin B with 5-nitro-8-hydroxyquinoline and the dilution of the complex, the enzyme inhibition was reduced to 21.7 ± 2.2 % which is in concordance with the expected inhibition for 5-nitro-8-hydroxyquinoline considering the Hill coefficient of inhibitor (calculated value of inhibition is 15 %). The inhibition did not significantly change even after prolonged incubation of cathepsin B with the inhibitor: 25.2 ± 3.5 % after 15 min and 27.6 ± 3.2 % after 30 min (Figure 1A).

Second, the reversibility of 5-nitro-8-hydroxyquinoline was also studied with the active site probe DCG-04. DCG-04 is a derivative of an irreversible epoxysuccinic inhibitor of cysteine proteases E-64 with an additional biotin moiety which enables detection of covalently modified proteases with labelling intensity correlating with enzymatic activity (Greenbaum D, Medzihradszky KF, Burlingame A, Bogyo M: Epoxide electrophiles as activity-dependent cysteine protease profiling and discovery tools. Chem Biol 2000, 7: 569-81.). 1 µg of recombinant cathepsin B in 50 mM Tris, pH 5.5 containing 1 mM DTT, 5 mM MgCl₂ and 250 mM sucrose was incubated with 0.1, 1, 10 in 50 µM 5-nitro-8-hydroxyquinoline (diluted from 10 mM DMSO stocks) for 1 h at room temperature. Samples were then labelled with 2 µM DCG-04 for an additional h at room temperature. After boiling in reducing sample buffer for 10 min samples were analyzed by 12.5 % SDS-PAGE and transferred to a nitrocellulose membrane. The latter was then blocked with 0.5 % Tween 20 in PBS overnight at 4 °C and incubated with 1 µg/ml streptavidin-horseradish peroxidase in 0.2 % Tween 20 in PBS for 1 h at room temperature. The membrane was washed five times with 0.2 % Tween 20 in PBS and the spots on the membrane were visualized with 0.5 mg/ml 3,3'-diaminobenzidine and 0.0005 % (v/v) H₂O₂ in 50 mM Tris, pH 7.5. CA-074, an irreversible cathepsin B inhibitor was used as a positive control.

The intensity of spots obtained with 5-nitro-8-hydroxyquinoline did not differ significantly from the DMSO control confirming the results obtained in the previous experiment that 5-nitro-8-hydroxyquinoline is a reversible inhibitor of cathepsin B (Figure 1B). When CA-074 was used as a positive control it caused a concentration dependent loss of spot intensity as expected.

Third, reversibility of 5-nitro-8-hydroxyquinoline was also checked with mass spectrometry. Cathepsin B (4.4 µM) was activated in the activation buffer for 5 min at 37 °C. Afterwards, an equimolar amount of the inhibitor was added to the enzyme and incubated for 1 h at room temperature. The mixture was dialyzed against water five times using Microcon Centrifugal Filter Devices YM-10 to avoid salt interferences and to remove unbound molecules of the test compound. Samples were then prepared by adding an equal volume of acetonitrile containing 0.1 % formic acid and analyzed with ESI-MS on an AutoSpec Q instrument. As a positive control CA-074 was used.

Molecular weight of cathepsin B was determined to be 28158 Da using ESI-MS and did not alter after incubation with 5-nitro-8-hydroxyquinoline confirming the reversible nature of the inhibitor. As expected in the case of CA-074 the mass of the enzyme changed for 384 Da, i.e. the mass of the bound fragment of the inhibitor.

### Cytotoxicity of 5-nitro-8-hydroxyquinoline on MCF-10A neoT cell line

A MTS assay was used to assess the cytotoxic effect of 5-nitro-8-hydroxyquinoline on MCF-10A neoT cells. 5 × 10⁴ cells in 100 µl of medium were added to a single well of a 96-microtiter plate to which a 100 µl of 5-nitro-8-hydroxyquinoline solution (twice the final concentration) was added. After 24 h incubation the cells were washed and the medium was replaced with 100 µl of fresh medium to which a 20 µl of MTS was added. After 2 h incubation the absorbance was measured at 490 nm. The experiment was performed in quadruplicates and DMSO was used as control.

10 µM concentration of 5-nitro-8-hydroxyquinoline was shown to be non-toxic on MCF-10A neoT cells (Figure 2A) using an MTS assay. This concentration was then used in the following functional tests on the MCF-10A neoT cell line.

### Inhibition of MCF-10A neoT cell line tumour invasion by 5-nitro-8-hydroxyquinoline

To assess the ability of 5-nitro-8-hydroxyquinoline to inhibit tumour cell invasion two independent experiments were used. First, an *in vitro* invasion model using Millicell Cell culture inserts (12 mm polycarbonate inserts with 12 µm pores) was performed. The bottom sides of 24-wells were coated with 100 µl of fibronectin from bovine plasma (25 µg/ml) and left in a laminar hood to dry. The upper side of Millicell inserts was coated with 100 µl of Matrigel (0.75 mg/ml in serum free medium) and incubated for 1 h at 37 °C. The inserts were then placed in with fibronectin coated wells. The upper compartments of the inserts were filled with 200 µl MCF-10A neoT cell suspension (7.5x10⁵/ml) containing 5-nitro-8-hydroxyquinoline (10 µM) in serum free medium and the bottom compartments were filled 500 µl of medium containing 10 µM 5-nitro-8-hydroxyquinoline and 10 % fetal calf serum. After 24 h incubation the bottom sides of wells were washed with 500 µl of PBS and the cells that were attached were detached by 0.05 % trypsin and 0.02 % EDTA in PBS. Detached cells were centrifuged for 5 min at 2000 rpm and were suspended in 100 µl of complete medium and transferred to wells of a 96-well plate to which 20 µl of MTS was added. After a 2 h incubation period the absorbance was measured at 490 nm. The experiment was carried out in triplicates and DMSO was used as control. The percentage of cell invasion was calculated as the ratio between the invading cells and the total cell population.

5-nitro-8-hydroxyquinoline significantly inhibited tumour invasion of MCF-10A neoT cell line *in vitro* (40.7 % compared to DMSO control) (Figure 2B).

The ability of 5-nitro-8-hydroxyquinoline to inhibit tumour cell invasion was also tested in a three-dimensional *in vitro* invasion model based on spheroids. Multicellular tumour spheroids were prepared according to hanging drop method (Kelm JM, Timmins NE, Brown CJ, Fussenegger M, Nielsen LK: Method for generation of homogeneous multicellular tumour spheroids applicable to a wide variety of cell types. *Biotechnol Bioeng* 2003, **83**:173-80.). Drops (20 µl) of MCF-10A neoT cell suspension (2.5×10/ml) were placed onto the lids of 10 cm dishes which were inverted over dishes containing 10 ml of PBS. Upon inversion of the tray the cells accumulated at the free liquid-air interface in this way forming spheroids after sufficient sedimentation time, in our case 6 days. Wells of a 24-well plate were filled with 400 µl of Matrigel (5mg/ml) containing the 5-nitro-8-hydroxyquinoline at the final concentration of 10 µM. After 15 min incubation at 37 °C 1 ml of medium with the test compound (10 µM) was placed on top of Matrigel. Spheroids were then carefully plated into the wells and incubated at 37 °C in a humidified atmosphere containing 5 % CO₂. Images of spheroids were obtained using an Olympus IX 81 motorized inverted microscope and CellR software.

5-nitro-8-hydroxyquinoline significantly inhibited tumour invasion of MCF-10A neoT cells in a three-dimensional *in vitro* invasion model. In the control experiment cells at the border of the spheroid invaded the Matrigel forming a corona around the original spheroid. 5-nitro-8-hydroxyquinoline completely abrogated the invasive activity at the edge of the spheroid.

### Inhibition of DQ-collagen IV degradation by 5-nitro-8-hydroxyquinoline

5-nitro-8-hydroxyquinoline was also tested for the ability to inhibit intracellular and extracellular degradation of DQ-collagen IV. First, inhibitory effect was observed using fluorescence microscopy. Wells of precooled Lab-Tek™ Chambered Coverglass (Nalge Nunc International) were coated with 25 µg/ml of the quenched fluorescent substrate DQ-collagen IV suspended in 40 µl of 100 % Matrigel for 10 min at 4°C. DQ-collagen IV/Matrigel matrix was allowed to polymerize for 40 min at 37°C. 400 µl of MCF-10A neoT cell suspension (1.5x10⁵/ml) in growth medium containing 2 % Matrigel and 10 µM of 5-nitro-8-hydroxyquinoline were plated onto gelled Matrigel. After 24 h incubation at 37°C with 5 % CO₂ the samples were monitored for fluorescent degradation products using Olympus IX 81 motorized inverted microscope and CellR software. CA-074 and CA-074Me were used as positive controls.

In a fluorescence microscopy based experiment 5-nitro-8-hydroxyquinoline inhibited both intracellular and extracellular DQ-collagen IV degradation. The latter was comparable to the inhibition caused by two cathepsin B irreversible inhibitors CA-074 and CA-074Me.

Two experiments were set to differentiate between inhibition of intracellular and extracellular DQ-collagen IV degradation. Inhibition of extracellular DQ-collagen IV degradation was observed by monitoring the fluorescence intensity of degraded DQ-collagen IV in the medium in which MCF-10A neoT cells were grown. 5 × 10⁴ MCF-10A neoT cells were plated into wells of a 96-well black plate and were allowed to attach over night. Cells were then incubated with 100 µl PBS containing 5-nitro-8-hydroxyquinoline, CA-074 or CA-074Me (10 µM) and DQ-collagen IV (5 µg/ml) for 3 h. Afterwards, the 80 µl of PBS was transferred into the empty wells and the fluorescence intensity was measured at 495 nm ± 5 nm excitation and 515 nm ± 5 nm emission wavelengths on Tecan Safire^{2™}. The experiment was performed in 8 parallels and DMSO was used as a control.

5-nitro-8-hydroxyquinoline significantly inhibits extracellular DQ-collagen IV degradation (35.3 %) which is comparable to the inhibition of two irreversible inhibitors of cathepsin B CA-074 (31.1 %) and CA-074Me (33.8 %) (Figure 3A).

Inhibition of intracellular DQ-collagen IV degradation was observed by flow cytometry by a shift in fluorescence intensity. 5 × 10⁴ MCF-10A neoT cells in complete medium were plated into the wells of a 24-well plate and allowed to attach over night. Afterwards, the medium was replaced with 500 µl of serum free medium with 5-nitro-8-hydroxyquinoline, CA-074 or CA-074Me (50 µM). After 2 h incubation the medium was again replaced with serum free medium containing inhibitors (50 µM) and DQ-collagen IV (5 µg/ml). DMSO was used as a control. For the experiment only viable cells were selected using propidium iodide. The measurements were performed on a FACS Calibur instrument (Beckton Dickinson, Inc.).

5-nitro-8-hydroxyquinoline significantly inhibits intracellular DQ-collagen IV degradation (Figure 3B). The latter is comparable to the inhibition of CA-04Me, a cell permeable cathepsin B irreversible inhibitor which was used as a positive control. CA-074, a non-permeable cathepsin B inhibitor only slightly inhibited intracellular DQ-collagen IV degradation.

### References

Dubin G: Proteinaceous cysteine protease inhibitors. Cell Mol Life Sci 2005, 62(6):653-69.
http://merops.sanger.ac.uk/
Frlan R, Gobec S: Inhibitors of cathepsin B. Curr Med Chem 2006, 13(19):2309-27.
Turk B: Targeting proteases: successes, failures and future prospects. Nat Rev Drug Discov 2006, 5(9):785-99.
Smith JH, Simons C: Proteinase and peptidase inhibition: recent potential targets for drug development. London, New York: Taylor & Francis, 2002. ISBN 0-415-27349-8.
Zeng GZ, Pan XL, Tan NH, Xiong J, Zhang YM: Natural biflavones as novel inhibitors of cathepsin B and K. Eur J Med Chem 2006, 41 (11):1247-52.
Barrett AJ, Kembhavi AA, Brown MA, Kirschke H, Knight CG, Tamai M, Hanada K: L-trans-Epoxysuccinyl-leucylamido(4-guanidino)butane (E-64) and its analogues as inhibitors of cysteine proteinases including cathepsins B, H and L. Biochem J 1982, 201:189-98.
Murata M, Miyashita S, Yokoo C, Tamai M, Hanada K, Hatayama K, Towatari T, Nikawa T, Katunuma N: Novel epoxysuccinyl peptides. Selective inhibitors of cathepsin B, in vitro. FEBSLett 1991, 280(2):307-10.
Towatari T, Nikawa T, Murata M, Yokoo C, Tamai M, Hanada K, Katunuma N: Novel epoxysuccinyl peptides. A selective inhibitor of cathepsin B, in vivo. FEBS Lett 1991, 280(2):311-5.
Buttle DJ, Murata M, Knight CG, Barrett AJ: CA074 methyl ester: a proinhibitor for intracellular cathepsin B. Arch Biochem Biophys 1992, 299(2):377-80.
Montaser M, Lalmanach G, Mach L: CA-074, but not its methyl ester CA-074Me, is a selective inhibitor of cathepsin B within living cells. Biol Chem 2002, 383(7-8):1305-8.
Premzl A, Zavasnik-Bergant V, Turk V, Kos J: Intracellular and extracellular cathepsin B facilitate invasion of MCF-10A neoT cells through reconstituted extracellular matrix in vitro. Exp Cell Res 2003, 283(2):206-14.
Schenker P, Alfarano P, Kolb P, Caflisch A, Baici A: A double-headed cathepsin B inhibitor devoid of warhead. Protein Sci 2008; 17(12):2145-55.

## Claims

1. Use of compound having general formula **(I),** 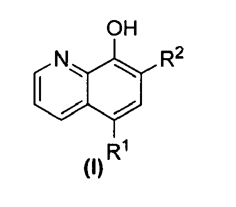 wherein:
R¹ is -H, -NO₂, C₁₋₆ branched or linear alkyl optionally substituted with one or more
substituents independently selected from the group of R⁵, -NO₂, -NH₂, 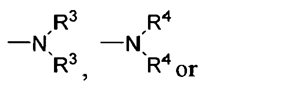 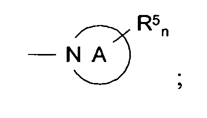 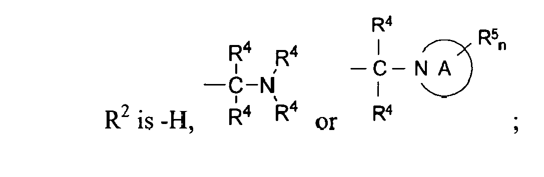 wherein A is a saturated or an unsaturated 3 to 8-membered heterocycle optionally comprising one or more further heteroatoms selected from N, O, S, and P, the heterocycle being optionally substituted with one or more substituents independently selected from R⁵, wherein n = 0 to 5;
R³ is -H, C₁₋₆ branched or linear alkyl optionally substituted with one ore more substituents independently selected from R⁵, C₃-C₆ cycloalkyl optionally substituted with one ore more substituents independently selected from R⁵;
R⁴ is -H; C₁₋₆ branched or linear alkyl optionally substituted with one ore more substituents independently selected from R⁵ and optionally comprising one or more heteroatoms selected from N, S, O, P and optionally substituted with -N(R³)_{2,} C₃-C₆ cycloalkyl optionally comprising one or more heteroatoms selected from N, S, O, P and optionally substituted with -N(R³)_{2,} aryl optionally substituted with one ore more substituents independently selected from R⁵, heteroaryl optionally substituted with one ore more substituents independently selected from R⁵;
R⁵ is -H, halo, -NH₂, -OH, -CN, -NO₂, -COOH, -CONR⁴R⁴, -SO₂NR⁴R⁴, -SO₂R⁴, -CF₃, -NHSO₂R⁴, -NHCOR⁴, C₁₋₆ branched or linear alkyl, C₁₋₆ alkoxy, -SR⁴;
wherein multiple occurrences of any of R³, R⁴ and R⁵ in the structure means that the respective residues are independently selected from the respective groups defined above;
or a pharmaceutically acceptable salt, hydrate or solvate thereof;
as an inhibitor of a cysteine protease.

2. Use according to claim 1, wherein the cysteine protease is a cathepsin B protease.

3. Use according to claim 1 or 2, wherein said inhibition is *ex vivo.*

4. Use according to any one of claims 1 to 3, wherein R¹ is selected from R⁵, - NH₂ and -NO₂.

5. Use according to any one of claims 1 to 4, wherein R¹ is -NO₂.

6. Use according to any one of claims 1 to 5, wherein R² is -H or

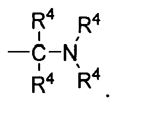

7. Use according to any one of claims 1 to 5, wherein R² is morpholinomethyl, pyrrolidinomethyl, (4-methylpiperazin-1-yl)methyl, (4-hydroxypiperidin-1-yl)methyl, (4-methypiperidin-1-yl)methyl, (4-carboxypiperidin-1-yl)methyl, or (3-hydroxypiperidin-1-yl)methyl.

8. A compound having general formula (I), 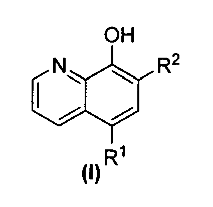 wherein:
R¹ is -H, -NO₂, C₁₋₆ branched or linear alkyl optionally substituted with one or more
substituents independently selected from the group of: R⁵, -NO₂, -NH₂, 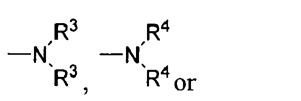 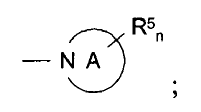 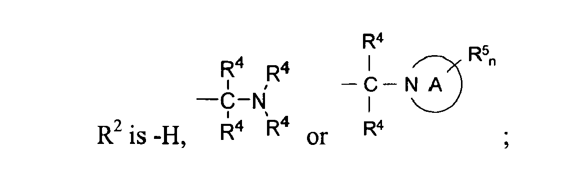 wherein A is a saturated or an unsaturated 3 to 8-membered heterocycle optionally comprising one or more further heteroatoms selected from N, O, S, or P; the heterocycle being optionally substituted with one or more substituents independently selected from R⁵ and n = 0 to 5;
R³ is -H, C₁₋₆ branched or linear alkyl optionally substituted with one ore more substituents independently selected from R⁵, C₃-C₆ cycloalkyl optionally substituted with one ore more substituents independently selected from R⁵;
R⁴ is -H; C₁₋₆ branched or linear alkyl optionally substituted with one ore more substituents independently selected from R⁵ and optionally comprising one or more heteroatoms selected from N, S, O, P and optionally substituted with -N(R³)_{2;} C₃-C₆ cycloalkyl optionally comprising one or more heteroatoms selected from N, S, O, P and optionally substituted with -N(R³)_{2;} aryl optionally substituted with one ore more substituents independently selected from R⁵; heteroaryl optionally substituted with one ore more substituents independently selected from R⁵;
R⁵ is -H, halo, -NH₂, -OH, -CN, -NO₂, -COOH, -CONR⁴R⁴, -SO₂NR⁴R⁴, -SO₂R⁴, -CF₃, -NHSO₂R⁴, -NHCOR⁴, C₁₋₆ branched or linear alkyl, C₁₋₆ alkoxy, -SR⁴;
wherein multiple occurrences of any of R³, R⁴ and R⁵ in a single structure means that the respective residues are independently selected from the members of the respective groups;
or a pharmaceutically acceptable salt, hydrate or solvate thereof;
for use as a medicament.

9. Compound of claim 8, wherein said medicament is useful in the treatment of a disease in a patient, wherein said disease is associated with an abnormal activity of a cysteine protease in said patient, preferably with an abnormal activity of cathepsin B protease in said patient.

10. Compound of claim 8 or 9, wherein said disease is cancer, rheumatoid arthritis, osteoarthritis, osteoporosis, pancreatitis, an immune disease, a neurodegenerative disease or Alzheimer's disease.

11. Compound of any one of claims 8 to 10, wherein said patient is a human or a mammal.

12. Compound of any one of claims 8 to 11, wherein R¹ is R⁵, -NH₂ or -NO₂.

13. Compound of any one of claims 8 to 12, wherein R¹ is -NO₂.

14. Compound of any one of claims 8 to 13, wherein R² is -H or

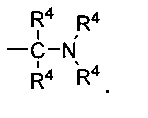

15. Compound of any one of claims 8 to 14, wherein R² is morpholinomethyl, pyrrolidinomethyl, (4-methylpiperazin-1-yl)methyl, (4-hydroxypiperidin-1-yl)methyl, (4-methypiperidin-1-yl)methyl, (4-carboxypiperidin-1-yl)methyl, or (3-hydroxypiperidin-1-yl)methyl.
